# EUROPEAN PATENT APPLICATION

(11) **EP 3 922 097 A1**
(43) Date of publication of application: **15.12.2021**
(21) Application number: 20178770.2
(22) Date of filing: 08.06.2020
(51) Int. Cl.: A01K 61/00, A01G 33/00, C12M 1/00, C12N 1/12

(54) **PROCESS AND APPARATUS FOR CLOSED-LOOP MULTITROPHIC AQUACULTURE**

(71) Applicant: Blue Planet Ecosystems GmbH, 2700 Wiener Neustadt (AT)
(72) Inventor: SCHMITZBERGER, Paul, 2700 Wiener Neustadt (AT); SCHMITZBERGER, Georg, 2700 Wiener Neustadt (AT); DANIELE, Thomas, 2700 Wiener Neustadt (AT)
(74) Representative: SONN Patentanwälte OG

(57) **Abstract**

The invention provides a process for the production of seafood in a closed-loop multitrophic aquaculture system comprising the steps of: (A) culturing phytoplankton in a medium, wherein said medium is irradiated with light; (B) transferring such cultured phytoplankton to a medium for culturing zooplankton; (C) transferring such cultured zooplankton to a medium for culturing seafood; and (D) transferring a part of the medium of step (C) as nutritient to the medium of step (A), thereby creating a closed loop, wherein steps (A) to (D) are carried out in a closed system, in which free gas exchange with the environment is prevented. The invention further provides an apparatus for carrying out this process.

## Description

The present invention relates to the field of multitrophic aquaculture systems.

With the population growth, the quantity of animal protein which will be necessary to satisfy the demand is projected to double by 2050. The production of animal protein thus puts significant pressure on agriculture and aquaculture systems and consequently on planetary health. Any agricultural system needs space where plants/crops trap solar energy and transform it into chemical energy in the form of fat, proteins, carbohydrates, etc., that human end-consumers use for food. However, the availability of arable land that meets the biological requirements for these crops is rapidly depleted due to climate change and overuse. This trend, in combination with the fact that only a small fraction of the solar energy reaching Earth's surface can be used for food production, makes agricultural land an extremely valuable commodity and a strategic resource for entire nations. The situation will further deteriorate due to climate change.

A significant portion of the total agricultural production, especially plant-based proteins from soybeans, is used in the production of animal protein. The fastest growing sector in animal-protein production is aquaculture. Aquaculture is developing quickly and has become the primary source of seafood, producing 53% of the consumed fish in 2016. Because of this rapid growth, the sector is beginning to face many environmental challenges that will make this type of farming more expensive and complicated in the next decades.

The first challenge is the negative environmental impact caused by intensive aquaculture farming operations. This negative environmental impact makes it harder for new aquaculture projects to acquire farming licenses from local governments. This is limiting the space on which additional aquaculture facilities can be conducted. The second challenge is related to the use of fish meals which are mostly made of crops, livestock co-products like blood meal as well as fishmeal and fish oil harvested from marine fisheries. The development of intensive aquaculture will thus increase the agricultural demand and suffer from growing prices.

Integrated multitrophic aquaculture (IMTA) has been proposed as a more environmentally friendly solution to seafood demand. IMTA refers to the co-culture of different species belonging to different trophic levels. Typically, IMTA systems provide the by-products, including waste, from one aquatic species as inputs for another. Species at the lower trophic level, e.g. plants or invertebrates, may use waste products from the higher trophic species as nutrients. The lower trophic species can be harvested in addition to the fish to give the farmer more revenue, or be fed back to the fish.

However, despite all developments in this area, new and improved multitrophic aquaculture systems that address at least some of the disadvantages of existing systems are needed. In particular, systems with high efficiency that can be flexibly deployed in different environments are required. It is an object of the present invention to provide such systems.

Therefore, the present invention provides a process for the production of seafood in a closed-loop multitrophic aquaculture system comprising the steps of:
(A) culturing phytoplankton in a medium, wherein said medium is irradiated with light;
(B) transferring such cultured phytoplankton to a medium for culturing zooplankton;
(C) transferring such cultured zooplankton to a medium for culturing seafood; and
(D) transferring a part of the medium of step (C) as nutritient to the medium of step (A), thereby creating a closed loop,
wherein steps (A) to (D) are carried out in a closed system, in which free gas exchange with the environment is prevented.

In another aspect, the invention provides an apparatus for carrying out the process according to the invention, comprising
- a phytoplankton unit for culturing phytoplankton,
- a zooplankton unit for culturing zooplankton,
- a seafood unit for culturing seafood, and
- a controller that, preferably automatically, controls the transfer of contents from the phytoplankton unit to the zooplankton unit, from the zooplankton unit to the seafood unit, and from the seafood unit to the phytoplankton unit,
wherein said units are interconnected containers that form a closed system, in which free gas exchange with the environment is prevented.

The invention thus provides a multitrophic aquaculture system that replicates aquatic ecosystems in order to convert CO₂ and light into organic, chemically unimpaired seafood using phyto- and zooplankton as intermediary stages. In the phytoplankton unit, phytoplankton such as microalgae can be grown. The phytoplankton can be cultured under optimal lighting conditions and be harvested on a continuous basis and pumped into the zooplankton unit. Therein, zooplankton can be grown under optimal conditions, using the phytoplankton as food. The biomass grown in this way can be transferred to the seafood unit, in which different species of fish and crustaceans can be grown. The wastewater and waste products of the seafood can be fed back to the phytoplankton unit, e.g. through a filtration- and conditioning system, and be used as growth medium for the phytoplankton, thus closing the cycle.

Known systems for multitrophic aquaculture are typically open systems, in which there is free and uncontrolled gas exchange with the environment (i.e. with the atmosphere). Such systems are highly dependent on environmental conditions. Changes in weather, humidity, pollution, contamination and other environmental influences can strongly impact growth conditions in the individual trophic levels, which can lead to a mismatch of growth levels of the various species and to strong variations in overall output and efficiency.

In contrast thereto, the present invention provides a closed system for multitrophic aquaculture, in which free gas exchange with the environment is prevented. This has a number of advantages. Importantly, the system is much less affected by environmental influences. For this reason, it can be operated more stably and be controlled much more precisely, which leads to a higher overall efficiency and output. The system is in particular less sensitive to changes in local environmental conditions, such as changes in weather, seasons, etc. For similar reasons, the system can also be advantageously used in environments that traditional open systems are less suitable for, e.g. deserts, cities, abandoned industrial plants and regions severely affected by climate change or pollution. It is furthermore possible to operate the system without the use of external water, which further adds to its environmental benefits and flexibility of deployment.

Moreover, providing a closed system for multitrophic aquaculture also has advantages for the efficiency of the process itself. Oxygen that is produced as a by-product in the phytoplankton unit can be used for providing optimal growth conditions in the zooplankton and seafood units instead of being lost to the environment. In a similar way, CO₂ produced in the zooplankton and seafood units can be used in the phytoplankton unit and be converted into biomass instead of being expelled to the environment. This leads to both a high efficiency, low power consumption and a low environmental impact of the inventive process.

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

In the following, all aspects of the invention are described together; preferred embodiments apply to the inventive process and to the inventive apparatus alike. For instance, any preferred embodiment of the phytoplankton, zooplankton and/or seafood unit comprised in the inventive apparatus also applies to the inventive process in the sense that it is preferred that the phytoplankton, zooplankton and/or seafood are cultured in the respective units.

Unless specified otherwise, all parameters as used herein correspond to parameters at IUPAC SATP-conditions ("Standard Ambient Temperature and Pressure"), in particular a temperature of 25 °C and a pressure of 101.300 Pa. Percentages (%) as used herein correspond to weight per volume (w/v) unless specified as weight per weight (w/w) or otherwise.

In the context of the present invention, the "closed system, in which free gas exchange with the environment is prevented" is understood as a system, in which there is no uncontrolled in- and outflow of gases into and out of said system. This does not, however, exclude controlled inflow or outflow of gases, e.g. controlled aeration or the possibility of excess gases leaving the system through valves or other means for relieving excess pressure. Preferably, the system is a system that can be sealed so that there is no gas exchange with the environment. Preferably, the system is sealed so that there is no gas exchange with the environment during at least a part of the inventive process, preferably during the full process. In a preferred embodiment of the inventive process, steps (A) to (D) are carried out in a sealed system, in which there is no gas exchange with the environment. In another preferred embodiment of the inventive process, there is controlled inflow of oxygen into the closed system, especially into the zooplankton and/or the seafood unit, during the process. The "closed system, in which free gas exchange with the environment is prevented" can also be referred to as a "sealable system, in which free gas exchange with the environment is prevented" or simply as a "system with no uncontrolled gas exchange with the environment". With regards to the inventive process, this means that it is preferred that there is no free (or uncontrolled) gas exchange between any of the media of steps (A), (B), and (C) with the environment (the atmosphere). With regards to the inventive apparatus this means that it is preferred that there is no free (or uncontrolled) gas exchange between the interconnected containers and the environment (the atmosphere).

In the context of the invention, "light" is to be understood as comprising any wavelength. Preferably the light is UV-light, visible light, or infrared light. It is preferred if the wavelength of the light is between 10 nm and 15.000 nm, preferably between 50 nm and 5.000 nm, more preferably between 100 nm and 2.000 nm, even more preferably between 200 nm and 1.000 nm. It is especially preferred if the light is sunlight. The medium in step (A) of the inventive process may be irradiated with natural or artificial light sources, either by actively irradiating the medium (e.g. with a LED light) or by positioning the system in such a way that it is irradiated by sunlight.

In a preferred embodiment of the inventive process, the transferring in steps (B), (C), and/or (D) is carried out automatically. With regards to the inventive apparatus, it is preferred if the controller controls the transfer of contents from the phytoplankton unit to the zooplankton unit, from the zooplankton unit to the seafood unit, and from the seafood unit to the phytoplankton unit automatically. In this context, "automatically" means that the transfer is triggered independently, without external (human) control, based on predetermined parameters. For example, the automatic transfer can be triggered by predetermined time intervals, environmental factors (e.g. measurements of temperature or light exposure), or combinations thereof. In a preferred embodiment, the automatic transfer is triggered by the growth levels of the cultured organisms. Preferably, the transfers in steps (B), (C), and/or (D) is triggered when the respective culture (phytoplankton for the transfer of step (B), zooplankton for step (C), seafood for step (D)) reaches a certain predetermined growth level. Preferably said growth levels are determined using video cameras, gas sensors, light sensors (e.g. for measuring light absorbance in the culture), chemical sensors, pH sensors or combinations thereof, especially video cameras.

In a preferred embodiment the medium of step (C) of the process is filtered before it is transferred to the medium of step (A), preferably using a drum filter and/or a cross-flow filtration system. The apparatus therefore preferably contains a filtration system, preferably comprising a drum filter and/or a cross-flow filtration system, for filtering the contents from the seafood unit before they are transferred to the phytoplankton unit.

In a preferred embodiment of the process according to the invention, the irradiation in step (A) is controlled by culture density of the phytoplankton. The denser the culture, the less light can penetrate the entire culture. Thus, by controlling the culture density, the average irradiation can be precisely controlled, which in turns makes it possible to control growth rates, e.g. in dependence on the growth rates of the other trophic levels. The culture density of the phytoplankton can in turn be controlled by controlling the amount and timing of the transfer of step (B) (of phytoplankton to the medium for culturing zooplankton).

The process according to the invention can be carried out with a wide variety of different species of phytoplankton, zooplankton and seafood. Preferably, the phytoplankton comprises *Chlorella* spp., especially *Chlorella vulgaris.* The zooplankton preferably comprises *Daphnia* spp. or *Cladocera* spp. Preferably the seafood is fish, preferably selected from zebrafish, blue-gills and tilapia. In another preferred embodiment, the seafood is shellfish, preferably shrimp, especially *Macrobrachium* shrimp. Shellfish have been found to lead to particularly advantageous results in the context of the present invention. It was found that when shellfish (shrimp) were used as seafood, there was less build-up of solid debris in the medium. Thus, an even more stable and efficient process, without the need of mechanical filtration, was obtained when shellfish were used as seafood.

In a preferred embodiment of the inventive process, the phytoplankton in step (A) is cultured in a medium with a surface-area-to-volume-ratio of at least 25 m⁻¹, preferably at least 30 m⁻¹, more preferably at least 35 m⁻¹, even more preferably at least 40 m⁻¹, yet even more preferably at least 45 m⁻¹, most preferably at least 50 m⁻¹. Preferably, the phytoplankton in step (A) is cultured in a medium with a surface-area-to-volume ratio between 25 m⁻¹ and 100 m⁻¹, preferably between 30 m⁻¹ and 90 m⁻¹, more preferably between 35 m⁻¹ and 80 m⁻¹, even more preferably between 40 m⁻¹ and 70 m⁻¹, yet even more preferably between 45 m⁻¹ and 65 m⁻¹, most preferably between 50 m⁻¹ and 60 m⁻¹. The same surface-area-to-volume ratios are preferred for the apparatus according to the invention; i.e. it is preferred if the phytoplankton unit (1) has a surface-area-to-volume-ratio as specified above. It was found that such high surface-area-to-volume ratios have unexpected advantages for temperature control. If the system is heated by sunlight during the day, excess energy can be efficiently dissipated during the night. The high surface area allows to both harvest large amounts of light energy and efficiently dissipate heat energy to the environment. This considerably lowers the risk of overheating the system.

It is especially preferred, if the phytoplankton unit comprises an array of multiple connected pipes or tubes, wherein the pipes or tubes are preferably arranged in parallel to each other. In this context, "multiple" means at least two, preferably at least 4, more preferably at least 10. Any suitable type of pipe or tube may be used; for instance, the pipes or tubes can have a round, rectangular, square, oval or other type of cross-section. The array can be a one-dimensional array (e.g. the pipes or tubes can be arranged in a plane) or, preferably, a two-dimensional array (e.g. the pipes or tubes can be arranged in the shape of a cuboid). In the context of the invention, it was found that such a tubular structure is advantageous both in terms of hydraulic and antifouling properties. Moreover, this type of structure is particularly well suited to ensure efficient harvesting of light energy and heat dissipation, as described herein above. The tubular array can act as a solar absorber during the day, and as a tube heat exchanger during the night. Preferably, the phytoplankton unit comprises walls consisting of glass, preferably borosilicate glass. Preferably, the pipes or tubes comprise glass, preferably borosilicate glass.

In a further preferred embodiment, said glass is switchable glass or smart glass. Switchable glass or smart glass is a glass or glazing whose light transmission properties can be altered by applied voltage, light or heat. Smart glass technologies include electrochromic, photochromic, thermochromic, suspended-particle, micro-blind, and polymer-dispersed liquid-crystal devices. Using such switchable glass or smart glass, the light transmission and thus the irradiation of the phytoplankton can be controlled.

It is preferred, if the volume of the phytoplankton unit is between 1 and 30 %, preferably between 3 and 15 %, even more preferably between 4 and 12 %, yet even more preferably between 5 and 10 %, most preferably between 6 and 8 % of the combined volume of the phytoplankton unit, the zooplankton unit and the seafood unit. In the context of the process according to the invention, the same preferred values apply to the volume of the medium in step (A) with respect the combined media of steps (A), (B) and (C). It has been found that these volumes enable a good balance of the growth of the phytoplankton with respect to the other trophic levels.

It is further preferred if the volume of the zooplankton unit is between 25 and 70 % of the combined volume of the phytoplankton unit, the zooplankton unit and the seafood unit, preferably between 35 and 60 %, more preferably between 40 and 55 %, especially between 44 and 50 %. With regards to the process according to the invention, the same preferred values apply to the medium in step (B) with respect the combined media of steps (A), (B) and (C). It is further preferred if the volume of the seafood unit is between 25 and 70 % of the combined volume of the phytoplankton unit, the zooplankton unit and the seafood unit, preferably between 35 and 60 %, more preferably between 40 and 55 %, especially between 44 and 50 %. With regards to the process according to the invention, the same preferred values apply to the medium in step (C) with respect the combined media of steps (A), (B) and (C).

In a preferred embodiment, the combined volume of the phytoplankton unit, the zooplankton unit and the seafood unit is between 60 m³ and 120 m³, preferably between 70 m³ and 100 m³, especially between 80 m³ and 90 m³, or multiples thereof. With regards to the process according to the invention, the same preferred values apply to the combined volume of the media of steps (A), (B) and (C). When the volumes are as specified, the dimensions of the apparatus can correspond to those of standard 40-foot shipping containers. Such shipping containers can be shipped internationally, without required special transports, which enables a flexible deployment of the inventive apparatus. Thus, the same infrastructure (cranes, trucks, trains, ships) that is used for moving shipping containers can be used for handling the apparatus. It is therefore preferred, that the dimensions of the apparatus correspond essentially to the dimensions of an ISO container, preferably a 40-foot ISO container (typically 12.2 m long, 2.4 m wide, 2.6 m high).

It is preferred if the phytoplankton unit has a volume of at least 0.25 m³, preferably at least 0.5 m³, more preferably at least 1 m³, even more preferably at least 2.5 m³, yet even more preferably at least 5 m³, most preferably at least 10 m³. Preferably the phytoplankton unit has a volume between 0.25 m³ and 50 m³, preferably between 0.5 m³ and 25 m³, more preferably between 1 m³ and 15 m³, even more preferably between 2.5 m³ and 10 m³, most preferably between 5 m³ and 7 m³. The same volumes are preferred for the medium in step (A) of the process according to the invention.

With regards to the zooplankton unit, it is preferred if said unit has a volume of at least 1 m³, preferably at least 5 m³, more preferably at least 10 m³ even more preferably at least 25 m³, most preferably at least 35 m³. Preferably, the zooplankton unit has a volume between 1 m³ and 250 m³, preferably between 5 m³ and 150 m³, more preferably between 10 m³ and 80 m³, even more preferably between 25 m³ and 60 m³, most preferably between 35 m³ and 45 m³. The same volumes are preferred for the medium in step (B) of the process according to the invention.

With regards to the seafood unit, it is preferred if said unit has a volume of at least 1 m³, preferably at least 5 m³, more preferably at least 10 m³ even more preferably at least 25 m³, most preferably at least 35 m³. Preferably, the zooplankton unit has a volume between 1 m³ and 250 m³, preferably between 5 m³ and 150 m³, more preferably between 10 m³ and 80 m³, even more preferably between 25 m³ and 60 m³, most preferably between 35 m³ and 45 m³. The same volumes are preferred for the medium in step (C) of the process according to the invention.

In a preferred embodiment of the apparatus according to the invention, the three units are stacked upon each other. This arrangement has the advantage that the units can be arranged in such a way that each unit obtains an optimal amount of light irradiation from the sunlight. Preferably, the phytoplankton unit is arranged closer to the light source (the sun or an artificial light source) than the zooplankton and seafood units. In this way, the phytoplankton unit can obtain a high amount of irradiation with sunlight while at the same time providing shade to the other two units, reducing the risk of overheating. Moreover, the arrangement in which the three units are stacked upon each other is advantageous for economical deployment in shipping containers. In an especially preferred embodiment, the phytoplankton unit is situated closer to the light source than the zooplankton unit, thus shading the latter, with the seafood unit being arranged below the zooplankton unit and thus farthest away from the light source.

Preferably, the apparatus according to the invention further comprises an artificial light source for irradiating the phytoplankton unit. Related thereto, it is preferred if the irradiation with light in step (A) of the inventive process is with light from an artificial light source. Preferably, the artificial light source is a LED. Advantageously, the artificial light source can provide irradiation in conditions of low or no ambient light; e.g. during dusk, dawn, or night. Moreover, the artificial light source can even out difference in weather conditions, e.g. when heavy cloud layers are present. In this way, a more stable continuous operation of the system can be ensured.

It is further preferred, if the apparatus comprises an artificial light source for irradiating the zooplankton and/or the seafood unit. With respect to the inventive process, it is preferred if the medium for culturing zooplankton and/or the medium for culturing seafood is irradiated using an artificial light source. This allows to adjust the circadian rhythm of the zooplankton and/or the seafood, which again allows a precise control of growth levels and a stable operation.

Preferably, the apparatus according to the invention further comprises a photovoltaic panel, which provides renewable electric energy to the apparatus and is arranged in such a way that it provides adjustable shade to the phytoplankton unit. The photovoltaic panel can thus shield the phytoplankton unit from excess irradiation in times of strong sunlight, preventing overheating of the system, and store the energy for times when natural light is low or not available (e.g. overcast skies or nighttime). The electric energy provided by the photovoltaic panel can thus be used for powering the artificial light source described above. Preferably, the apparatus further comprises an energy storage device, preferably a battery, for storing electric energy provided by the photovoltaic panel. Preferably the battery is a gel battery stack and/or a redox flow battery stack. Preferably the battery has a capacity of at least 10 kWh, more preferably at least 50 kWh, especially at least 100 kWh. In this way, the photovoltaic panel and the battery can together act as an energy buffer. In addition, an emergency power supply is available in case of grid failure. Thus, also for the inventive process, it is preferred if the irradiation with light in step (A) is with light from an artificial light source powered with electric energy from a photovoltaic panel, which is arranged in such a way that it provides adjustable shade to the medium in which the phytoplankton is cultured. In the context of the inventive process, the electric energy from the photovoltaic panel can be stored in a battery and used for powering the artificial light source, as described above for the inventive apparatus. Preferably, the photovoltaic panels are moveable so that the amount of shade can be adjusted during the operation of the system. Moveable photovoltaic panels have the additional advantage of facilitating self-cleaning, since rain can be used for removing dirt or dust from the phytoplankton unit. It is particularly preferred if the shade provided by the photovoltaic panel is automatically adjustable, for instance, if the photovoltaic panels are automatically rearranged depending on the intensity of irradiation by sunlight.

In a preferred embodiment of the inventive process, the phytoplankton and/or the zooplankton and/or the seafood is cultured under flow conditions. Preferably, all three trophic levels are cultured under flow conditions. With respect to the apparatus according to the invention, it is therefore preferred if it further comprises pumps to provide flow within the phytoplankton unit and/or the zooplankton unit and/or the seafood unit. Preferably, the apparatus comprises separate pumps for each of the three units. It is particularly preferred, if the flow in each unit can be controlled individually, i.e. independently from the flow in the other two units. Providing flow has the advantages of ensuring proper mixing of culture media, allowing flushing of the system, and dislodging of biofilms. Providing flow is particularly preferred for the seafood unit. Culturing the seafood under flow conditions has the advantage of providing physical exercise to the seafood, which can promote muscle development.

In a preferred embodiment of the invention, the apparatus further comprises heat exchangers to control the temperature inside the phytoplankton unit and/or the zooplankton unit and/or the seafood unit, wherein suitable tubing provides for temperature control in each unit by using excess heat from other unit(s). In this way, excess heat from some units is not lost to the environment but instead efficiently used for adjusting the temperature in other units.

It is further preferred if the apparatus further comprises a thermal reservoir. Such a reservoir can store thermal energy during warmer time periods, e.g. during the day, and heat the media in the individual units during colder time periods, e.g. during the night.

Since the phytoplankton can consume CO₂ and produce O₂, whereas the zooplankton and seafood can consume O₂ and produce CO₂ it is advantageous, if there is controlled gas exchange between the different units. Preferably, the apparatus therefore comprises conduits for gas exchange between the phytoplankton and the zooplankton unit and/or between the phytoplankton and the seafood unit. With regards to the inventive process, it is preferred if there is gas exchange between the medium in step (A) with the medium in step (B), and/or between the medium in step (A) with the medium in step (C).

Preferably, said gas exchange is facilitated by packed media aeration columns. In a preferred embodiment, the apparatus therefore comprises packed media aeration columns for this purpose. Packed media aeration columns can be ventilated tubes that are filled with a material with a high specific surface area. This large surface area helps in the formation of a thin water film that covers this media as a constant stream of water is introduced at the top and flows to the bottom. This significantly increases the gas-liquid interface where the gas exchange takes place. The thickness of the boundary layer between air and water is influenced by the (laminar) flow of the water and the velocity of the air. Air can be introduced at the bottom with fans to achieve a counter flow. The air- and water flow through the packed media aeration columns can be controlled in order to control the speed of gas exchange. In a preferred embodiment, the phytoplankton unit and/or the zooplankton unit and/or the seafood unit comprises a packed media aeration column. It is most preferred if all units comprise packed media aeration columns. In this way, imbalances from the consumption/production of O₂/CO₂ (over- or undersaturation of the culture media) can be quickly corrected.

It is further preferred, if the phytoplankton unit and/or the zooplankton unit and/or the seafood unit comprises gas sensors, in particular CO₂ and/or O₂ sensors. The data provided by such sensors can be used to control the gas exchange between the different units, e.g. by controlling the air- and water flow through the packed media aeration columns described above.

In another preferred embodiment, the apparatus further comprises one or more cameras for monitoring the seafood unit and/or the zooplankton unit and/or the phytoplankton unit. It is particularly preferred if it comprises at least one camera for monitoring the seafood unit. With regards to the inventive process, it is preferred if the cultured seafood, zooplankton, and/or phytoplankton are monitored using one or more cameras. It is most preferred if all units are monitored using one or more cameras. Such cameras allow monitoring of the growth levels of the cultured organisms, for instance to ensure that the growth is kept in an exponential phase. Preferably, the data obtained from the one or more cameras is used for triggering the automatic transfer of contents from the phytoplankton unit to the zooplankton unit, from the zooplankton unit to the seafood unit, and/or from the seafood unit to the phytoplankton unit.

In the seafood unit, the one or more cameras can advantageously be used for analysing the position and speed of individual animals. In addition, their color, growth, and/or breathing patterns can be analysed. From this information, many conclusions can be drawn about the well-being of the fish and the state of the ecosystem. It is possible to detect a sick fish, for instance, by noticing that its speed is lower than that of the rest of the fish population. It is also possible to come to that conclusion by the analysis of the breathing pattern or the position of the individual. A dead fish could also be detected using a similar method. This information is very valuable because fish populations in aquaculture can be sensitive to diseases. All these data can be used as input to control the preferably automatic transfer of contents between the different units, as well as other parameters, such as temperature, irradiation, or gas exchange.

The present invention is further illustrated by the following figure, without being limited thereto.

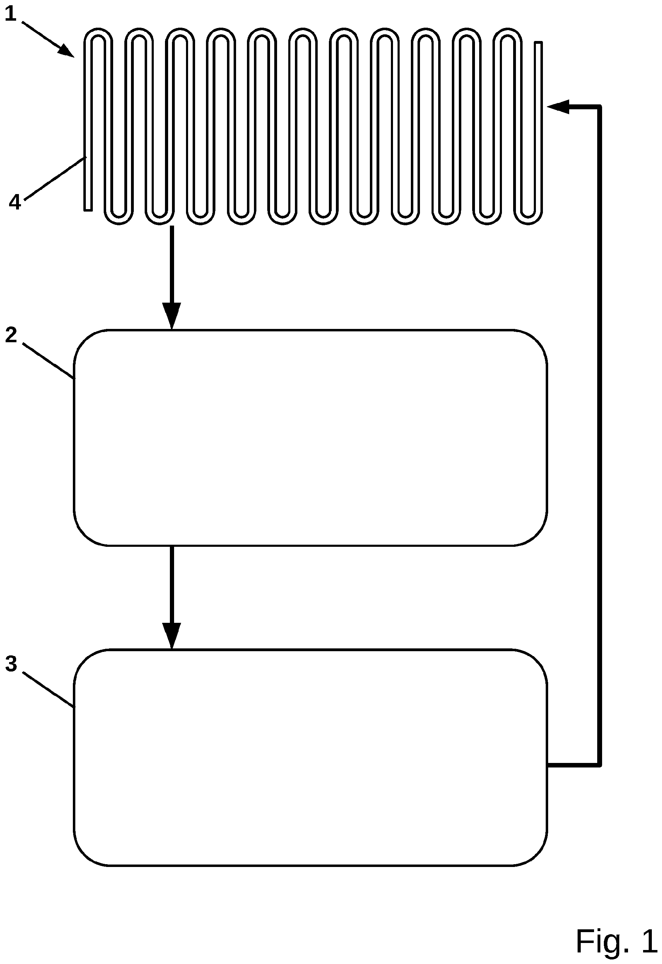

Figure 1 shows a schematic representation of a preferred embodiment of the apparatus according to the invention. The apparatus comprises a phytoplankton unit (1) for culturing phytoplankton, a zooplankton unit (2) for culturing zooplankton, a seafood unit (3) for culturing seafood, and a controller (not shown) that controls the transfer of contents from the phytoplankton unit (1) to the zooplankton unit (2), from the zooplankton unit (2) to the seafood unit (3), and from the seafood unit (3) to the phytoplankton unit (1). The units (1)-(3) are interconnected containers (connections only shown schematically) that form a closed system, in which free gas exchange with the environment is prevented. In the displayed preferred embodiment, the phytoplankton unit (1) comprises an array of multiple connected pipes or tubes (4) arranged in parallel to each other.

## Claims

1. A process for the production of seafood in a closed-loop multitrophic aquaculture system comprising the steps of:
(A) culturing phytoplankton in a medium, wherein said medium is irradiated with light;
(B) transferring such cultured phytoplankton to a medium for culturing zooplankton;
(C) transferring such cultured zooplankton to a medium for culturing seafood; and
(D) transferring a part of the medium of step (C) as nutritient to the medium of step (A), thereby creating a closed loop,
wherein steps (A) to (D) are carried out in a closed system, in which free gas exchange with the environment is prevented.

2. The process according to claim 1, wherein the phytoplankton in step (A) is cultured in a medium with a surface-area-to-volume-ratio of at least 25 m⁻¹.

3. The process according to claim 1 or 2, wherein the irradiation in step (A) is controlled by culture density of the phytoplankton.

4. The process according to any one of the preceding claims, for the production of shellfish, preferably shrimp.

5. An apparatus for carrying out the process according to any one of the preceding claims, comprising
- a phytoplankton unit (1) for culturing phytoplankton,
- a zooplankton unit (2) for culturing zooplankton,
- a seafood unit (3) for culturing seafood, and
- a controller that, preferably automatically, controls the transfer of contents from the phytoplankton unit (1) to the zooplankton unit (2), from the zooplankton unit (2) to the seafood unit (3), and from the seafood unit (3) to the phytoplankton unit (1),
wherein said units are interconnected containers that form a closed system, in which free gas exchange with the environment is prevented.

6. The apparatus according to claim 5, wherein the phytoplankton unit (1) has a surface-area-to-volume-ratio of at least 25 m⁻¹.

7. The apparatus according to claim 5 or 6, wherein the phytoplankton unit (1) comprises an array of multiple connected pipes or tubes (4), wherein the pipes or tubes are preferably arranged in parallel to each other.

8. The apparatus according to any one of claims 5 to 7, wherein the combined volume of the phytoplankton unit (1), the zooplankton unit (2) and the seafood unit (3) is between 80 m³ and 90 m³, or multiples thereof.

9. The apparatus according to any one of claims 5 to 8, further comprising an artificial light source, preferably a LED, for irradiating the phytoplankton unit (1).

10. The apparatus according to any one of claims 5 to 9, wherein the phytoplankton unit (1) is situated closer to the light source than the zooplankton unit (2), thus shading the latter, with the seafood unit (3) being arranged below the zooplankton unit (2) and thus farthest away from the light source.

11. The apparatus according to any one of claims 5 to 10, further comprising a photovoltaic panel, which provides renewable electric energy to the apparatus and is arranged in such a way that it provides adjustable shade to the phytoplankton unit (1).

12. The apparatus according to any one of claims 5 to 11, further comprising pumps to provide flow within the phytoplankton unit (1) and/or the zooplankton unit (2) and/or the seafood unit (3) .

13. The apparatus according to any one of claims 5 to 12, further comprising heat exchangers to control the temperature inside the phytoplankton unit (1) and/or the zooplankton unit (2) and/or the seafood unit (3), wherein suitable tubing provides for temperature control in each unit by using excess heat from other unit(s).

14. The apparatus according to any one of claims 5 to 13, wherein the phytoplankton unit (1) and/or the zooplankton unit (2) and/or the seafood unit (3) comprises a packed media aeration column.

15. The apparatus according to any one of claims 5 to 14, further comprising at least one camera for monitoring the seafood unit (3).
